(19) Europäisches Patentamt
European Patent Office
Office européen des brevets

(11) Veröffentlichungsnummer: **0 270 074 A2**

(12) **EUROPÄISCHE PATENTANMELDUNG**

(21) Anmeldenummer: 87117750.7

(22) Anmeldetag: 01.12.87

(51) Int. Cl.⁴: C07D 401/06 , A01N 43/50 , C07D 401/14 , C07D 403/06

(30) Priorität: 03.12.86 DE 3641343

(43) Veröffentlichungstag der Anmeldung: 08.06.88 Patentblatt 88/23

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Ehrhardt, Heinz, Dr.
Bergstrasse 21
D-8901 Rehling(DE)
Erfinder: Mildenberger, Hilmar, Dr.
Fasanenstrasse 24
D-6233 Kelkheim (Taunus)(DE)
Erfinder: Maier, Thomas, Dr.
Rauenthaler Weg 22
D-6000 Frankfurt am Main 71(DE)
Erfinder: Schaller, Rainer, Dr.
Thyssenstrasse 31
D-8906 Gersthofen(DE)
Erfinder: Sachse, Burkhard, Dr.
An der Ziegelei 30
D-6233 Kelkheim (Taunus)(DE)
Erfinder: Braun, Peter, Dr.
Pfarrer-Dorn-Strasse 13
D-6500 Mainz(DE)

(54) **Carbamoylimidazol-Derivate, Verfahren zu ihrer Herstellung und ihre Verwendung als Fungizide.**

(57) Verbindungen der Formel (I)

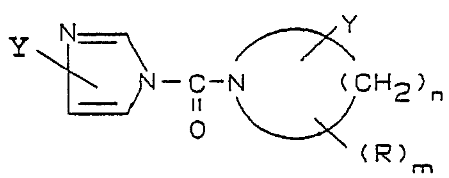

(I)

worin n eine Zahl von 2 bis 6, m = 1 oder 2,
R = $-OR^1$,

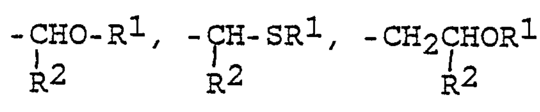

oder $-CH_2\ CH_2\ CH_2\ OR^1$,

EP 0 270 074 A2

$R^1$ = (subst.)-Alkyl, (subst.)-Alkenyl, C -Alkinyl, Cycloalkyl, Cycloalkenyl, ein aromatischer oder heteroaromatischer Rest, $R^2$ = H oder Alkyl und
Y = H, Alkyl, Phenyl oder Halogenphenyl sowie deren Metallsalzkomplexe besitzen vorteilhafte fungizide Wirkungen und eignen sich hervorragend zum Einsatz im Pflanzenschutz oder im technischen Bereich.

87117750.7

HOECHST AKTIENGESELLSCHAFT HOE 86/F 296 Dr.LO/AW

Beschreibung

## Carbamoylimidazol-Derivate, Verfahren zu ihrer Herstellung und ihre Verwendung als Fungizide

Die vorliegende Erfindung betrifft neue Carbamoylimidazol-Derivate, Verfahren zu ihrer Herstellung und ihre Verwendung als Pflanzenbehandlungsmittel.

Es wurde bereits offenbart, daß bestimmte Carbamoylderivate fungizide Aktivität besitzen (vgl. DE-OS 28 12 662). Ihre Wirkungsstärke, ihre Wirkungsbreite sowie ihre Verträglichkeit sind jedoch nicht ganz befriedigend.

Es wurde nun gefunden, daß neue Carbamoylderivate diese Nachteile nicht besitzen.

Die Erfindung betrifft daher Carbamoylimidazolderivate der Formel I

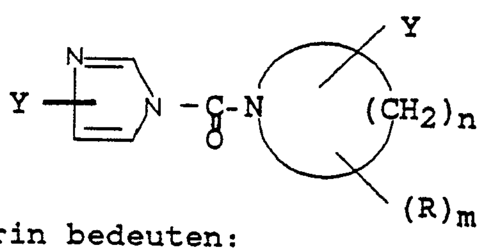

(I)

worin bedeuten:

n = 2, 3, 4, 5 oder 6,

m = 1 oder 2,

R = $-OR^1$, $-\underset{R^2}{CH}O-R^1$, $-\underset{R^2}{CH}-SR1$, $-CH_2\underset{R^2}{CH}OR^1$, oder $-CH_2CH_2CH_2OR^1$,

$R^1$= $(C_1-C_{10})$-Alkyl, $(C_3-C_{10})$-Alkenyl, die beide durch 1 bis 6 Fluor-, Chlor- oder Bromatome, Hydroxy, $(C_1-C_4)$-Alkoxy, Phenyl oder Phenoxy, die beide unsubstituiert oder durch Halogen, $(C_1-C_4)$Alkyl oder $(C_1-C_4)$Alkoxy substituiert sind, substituiert sein können, $C_3$-Alkinyl, $(C_3-C_6)$-Cycloalkyl, $(C_5-C_6)$-Cycloalkenyl, Phenyl, Benzyl, Benzhydryl, Trityl, Biphenyl, Phenoxyphenyl,

Phenylthiophenyl, Naphthyl, 1,2,3,4-Tetrahydronaphthyl, Indanyl, Fluorenyl, Pyridyl, Benzthiazolyl, Benzoxazolyl, Chinolinyl, Thiochromanyl, Benzofuranylmethyl, Chinolinylmethyl, Phenacyl, wobei die genannten Ringsysteme unsubstituiert oder mit 1 - 5 Substituenten aus der Gruppe Halogen, $(C_1-C_8)$-Alkyl, Halo$(C_1-C_8)$alkyl, $(C_3-C_8)$-Cycloalkyl, $(C_1-C_8)$-Alkoxy, Halo$(C_1-C_8)$alkoxy, $(C_3-C_8)$-Cycloalkyl-$(C_1-C_4)$alkyl, $(C_1-C_8)$Alkylthio, Halo$(C_1-C_4)$alkylthio, $(C_1-C_8)$-Alkylsulfinyl , $(C_1-C_8)$Alkylsulfonyl, $NO_2$, -CN, -CHO oder $(C_1-C_4)$Alkylcarbonyl substituiert sind; $(C_4-C_{10})$Alkadienyl,

$R^2$= H oder $(C_1-C_4)$Alkyl und

Y = unabhängig voneinander H, $(C_1-C_4)$Alkyl, Phenyl oder Halogenphenyl sowie deren Metallsalzkomplexe.

Die genannten Halo$(C_1-C_8)$alkyl oder Halo$(C_1-C_8)$alkoxy Reste enthalten insbesondere 1 bis 9 Fluor- oder Chloratome. Genannt seien beispielsweise $-CF_3$, $-C_2F_5$, $-CCl_3$, ω-Chloroctyl, $OCF_3$, Hexafluorpropoxy.

Bevorzugt sind die Verbindungen der Formel I, in denen n = 4 oder 5 und m = 1 oder 2, insbesondere 1 bedeutet. Besonders bevorzugt sind ferner Verbindungen der Formel I, in denen darüber hinaus R für $OR^1$, $CH_2OR^1$ oder $CH_2CH_2OR^1$ und Y für H steht, und $R^1$ Phenyl, Benzyl, Benzhydryl, Biphenyl, Pyridyl oder Chinolyl bedeutet, wobei die genannten Ringsysteme unsubstituiert oder mit 1 - 3 Substituenten aus der Gruppe F, Cl, Br, $(C_1-C_3)$-Alkyl, Halo$(C_1-C_3)$alkyl, $(C_1-C_3)$-Alkoxy, Halo$(C_1-C_3)$alkoxy, $(C_1-C_3)$-Alkylthio, $CF_3S$, $CF_3CH_2S$, $NO_2$, CN, CHO oder $-COCH_3$ substituiert sind, sowie deren Metallsalzkomplexe.

Die vorliegende Erfindung betrifft die Verbindungen (I) in Form der freien Basen oder von Metallsalzkomplexen. Genannt seien Komplexe mit Metallen der Gruppen Ib, IIb,

IVb, VII oder VIII des Periodensystems etwa Kupfer, Zink, Zinn oder Mangan. Die Herstellung derartiger Komplexe erfolgt nach allgemein üblichen Methoden.

Die Verbindungen (I) weisen mindestens ein asymmetrisches C-Atom auf und können daher als Enantiomere und Diastereomere auftreten. Die Erfindung umfaßt sowohl die reinen Isomeren als auch deren Gemische. Die Gemische von Diastereomeren können nach gebräuchlichen Methoden, zum Beispiel durch selektive Kristallisation aus geeigneten Lösungsmitteln oder durch Chromatographie an Kieselgel oder Aluminiumoxid in die Komponenten aufgetrennt werden. Racemate können nach üblichen Methoden in die Enantiomeren aufgetrennt werden, so zum Beispiel durch Salzbildung mit einer optisch aktiven Säure, Trennung der diastereomeren Salze und Freisetzung der reinen Enantiomeren mittels einer Base.

Gegenstand der Erfindung sind auch Verfahren zur Herstellung der Verbindungen der Formel I, dadurch gekennzeichnet, daß man

a) eine Verbindung der Formel (II)

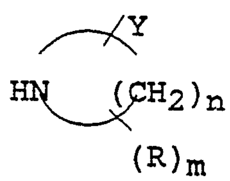

(II)

mit einer Verbindung der Formel (III)

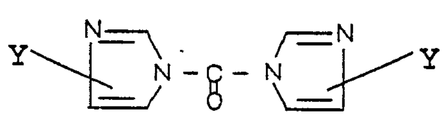

(III)

worin Y in beiden Imidazolresten vorzugsweise dieselbe Bedeutung besitzt, gegebenenfalls in Gegenwart von Säure-Acceptoren und inerten Lösungsmitteln, oder

b) eine Verbindung der Formel (IV)

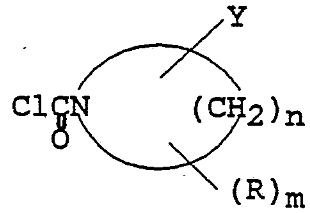 (IV)

mit einer Imidazolverbindung der Formel (V)

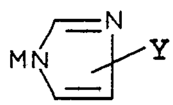 (V)

worin M = ein Alkalimetall, insbesondere Na oder K, bedeutet, umsetzt und die erhaltene Verbindung gegebenfalls in einen Metallsalzkomplex überführt. Die Verbindungen der Formel II sind zum Teil bekannt, s. Bull. Soc. Chim. Fr. (1947) 341, 344; Arch. Pharm. 292, (1959) 165, 167 oder können aus den entsprechenden Hydroxyalkylpiperidinen nach dem Fachmann bekannten Methoden hergestellt werden.

Als Beispiele seien genannt:
2-(2-Chlorbenzyloxymethyl)-piperidin, 2-(4-Trifluormethylphenoxymethyl)-piperidin, 2-[2-(4-Trifluormethylphenoxyethyl]-piperidin, 2-[3-(4-Trifluormethylphenoxy)-propyl]-piperidin,
4-(4-Trifluormethylphenoxy)-piperidin, 2-[2-Chlor-4-trifluormethyl-phenoxy-methyl]-pyrrolidin,
2-(4-Trifluormethylbenzyloxy-methyl)-piperidin,
2-(2,4,6-Trichlorphenoxy-methyl)-piperidin.

Die Verbindungen der Formel IV sind aus den Verbindungen der Formel I nach gängigen Methoden herstellbar. Die Verbindungen der Formeln III und V sind literaturbekannt und zum Teil im Handel erhältlich.

Das Verfahren zur Herstellung der Verbindungen der allgemeinen Formel (I) gemäß der Verfahrensvariante a) kann zweckmäßigerweise in einem inerten Lösungsmittel oder Verdünnungsmittel durchgeführt werden. Als solche

kommen in Betracht:
aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Ethylisobutylketon; Nitrile wie Acetonitril, Propionitril und Acrylnitril; Alkohole wie Methanol, Ethanol, Isopropanol, Butanol und Ethylenglykol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die Reaktionsvariante a) wird gegebenenfalls in Gegenwart eines Säure-Acceptors durchgeführt. Beispiele für solche Säure-Acceptoren umfassen die Hydroxide, Carbonate, Hydrogencarbonate und Alkoholate von Alkalimetallen, insbesondere von Na und K, oder tertiäre Amine wie Triethylamin, Diethylanilin und Pyridin.

Die Reaktionsvariante a) kann innerhalb eines breiten Temperaturbereichs durchgeführt werden. Im allgemeinen wird sie bei einer Temperatur zwischen etwa -20°C und dem Siedepunkt der Reaktionsmischung, vorzugsweise zwischen etwa 0°C und etwa 100°C, durchgeführt. Die Reaktion wird vorzugsweise unter normalem atmosphärischem Druck durchgeführt; jedoch ist es auch möglich, unter erhöhtem oder vermindertem Druck zu arbeiten.

Die Reaktionsvariante b) gemäß der vorliegenden Erfindung wird vorzugsweise in Gegenwart eines inerten Lösungsmittels oder Verdünnungsmittels durchgeführt. Es finden die unter a)

angegebenen Lösungsmittel oder Verdünnungsmittel Anwendung. Die Reaktionsvariante b) wird vorzugsweise in Gegenwart von Säure-Acceptoren durchgeführt. Beispiele für solche Säure-Acceptoren umfassen die Hydroxide, Carbonate, Hydrogencarbonate und Alkoholate von Alkalimetallen und tertiäre Amine wie Triethylamin, Diethylanilin und Pyridin.

Die Reaktionsvariante b) kann innerhalb eines breiten Temperaturbereichs durchgeführt werden, beispielsweise bei einer Temperatur zwischen etwa - 20° C und dem Siedepunkt der Reaktionsmischung, vorzugsweise zwischen 0° C und 100° C. Die Reaktionsvariante b) wird unter normalem Druck durchgeführt, jedoch ist es auch möglich, unter erhöhtem oder verminderten Druck zu arbeiten.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytopathogener Pilze, wie z.B. Piricularia oryzae, Echte Mehltauarten, Fusariumarten, Plasmopora viticola, Pseudo-peronospora cubensis, verschiedene Rostpilze und Pseudocercosporella herpotrichoides. Besonders gut werden Benzimidazol- und Dicarboximid-sensible und -resistente Botrytis cinerea-Stämme erfaßt.

Die Verbindungen der Formel I eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Gegenstand der vorliegenden Erfindung sind daher auch fungizide Mittel, die eine Verbindung der Formel I neben geeigneten Hilfsmitteln enthalten.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylenphenylsulfonate und Dispergiermittel, z.B. ligninsulfonsaures Natrium, 2,2'-dinaphthylmethan-6,6'-disulfonsaures Natrium, 2,2'-dibutylnaphthalinsulfonsaures Natrium oder auch oleoylmethyltaurinsaures Natrium enthalten. Ihre Herstellung erfolgt in üblicher Weise z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwandt werden: Alkylarylsulfonsaure Calziumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyglykolether Sorbitanfettsäureester, Polyoxyethylensorbitanfettsäureester oder Polyoxethylensorbitester.

Stäubemittel kann man durch Vermahlen des Wirkstoffes mit fein verteilten festen Stoffen, z.B. Talkum, natürlichen Tonen wie Kaolin, Bentonit, Poryphillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%; der Rest zu 100 Gew.-% besteht aus üblichen Formulierungsbestandteilen. Bei emulgierbaren Konzentraten kann die Wirkstoffkonzentration etwa 10 bis 80 Gew.-% betragen.

Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Die Aufwandmengen der erfindungsgemäßen Verbindungen variieren im allgemeinen zwischen 0,01 und 2,0 kg/ha, insbesondere zwischen 0,05 und 1,5 kg/ha.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen der Verbindungen der Formel I, insbesondere die der nachfolgenden Beispiele mit anderen Wirkstoffen, wie z.B. Insektiziden, Akariziden, Herbiziden, Düngemitteln, Wachstumsregulatoren oder Fungiziden können angewendet werden. Hierbei werden teilweise auch synergistische Wirkungssteigerungen erzielt.

Geeignete fungizide Kombinationspartner sind beispielsweise die Wirkstoffe
Imazalil, Prochloraz, Fenapanil, SSF105, Triflumizol, PP969, Flutriafol, BAY-MEB 6401, Propiconazol, Etaconazol, Diclobutrazol, Bitertanol, Triadimefon, Triadimenol, Fluotrimazol, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol, Bupirimate, Rabenzazole, Tricyclazole, Ofurace, Furalaxyl, Benalaxyl, Metalaxyl, Pencyuron, Oxadixyl, Cyprofuram, Dichlomezin, Probenazole, Fluobenzimine, Pyroxyfur, NK-483, PP-389, Pyroquilon, Hymexazole, Fenitropan, UHF-8227, Tolclofosmethyl, Ditalimfos, Edifenphos, Pyrazophos, Isoprothiolane, Cymoxanil, Dichloruanid, Captafol, Captan, Folpet, Tolylfluanid, Chlorothalonil, Etridiazol, Iprodione, Procymidon, Vinclozolin, Metomeclan, Myclozolin, Dichlozolinate, Fluorimide, Drazoxolon, Chinomethionate, Nitrothalisopropyl, Dithianon, Dinocap, Binapacryl, Fentinacetate, Fentinhydroxide, Carboxin, Oxycarboxin, Pyracarbolid, Methfuroxam, Fenfuram, Furmecyclox,

Benodanil, Mebenil, Mepronil, Flutolanil, Fuberidazole, Thiabendazole, Carbendazim, Benomyl, Thiofanate, Thiofanate-methyl, CGD-94240F, IKF-1216, Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cu-oxychlorid, Oxine-Cu, Cu-oxide, Schwefel, Fosetylaluminium,

Natrium-dodecylbenzolsulfonat,
Natrium-dodecylsulfat,,
Natrium-C13/C15-alkoholethersulfonat,
Natrium-cetostearylphosphatester,
Dioctyl-natriumsulfosuccinat,
Natrium-isopropylnaphthalinsulfonat,
Natrium-methylenbisnaphthalinsulfonat,
Cetyl-trimethyl-ammoniumchlorid,
Salze von langkettigen primären, sekundären oder tertiären Aminen,
Alkyl propylenamine,
Lauryl-pyridiniumbromid,
Ethoxilierte quaternierte Fettamine,,
Alkyl-dimethyl-benzyl-ammoniumchlorid und
1-Hydroxyethyl-2-alkyl-imidazolin.

Die Verbindungen der Formel I sowie die genannten Kombinationspartner stellen alle bekannte Wirkstoffe dar, die zum großen Teil in CH. R. Worthing, S.B. Walker, The Pesticide Manual, 7. Auflage (1983), British Crop Protection Council beschrieben sind. Verbindungen, für die Nummerncodes angegeben sind, besitzen folgende Strukturen:

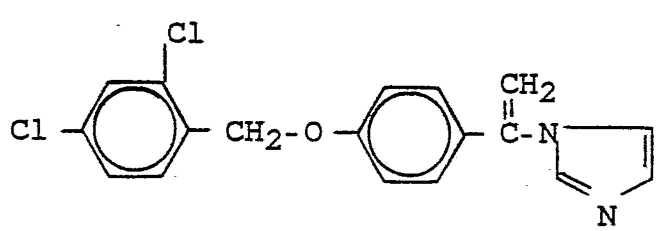

SS F 105

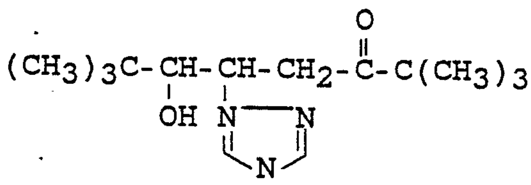

PP 969

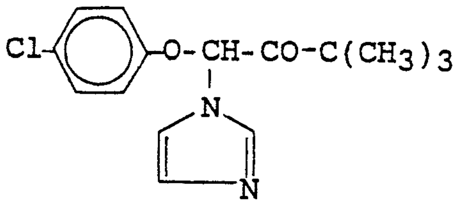

Bay-Meb
6401

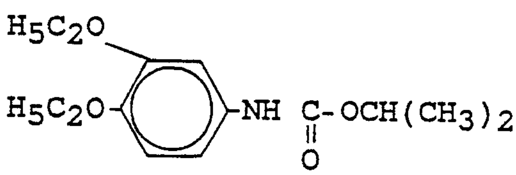

S - 32165

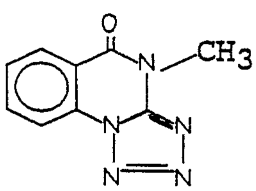

PP 389

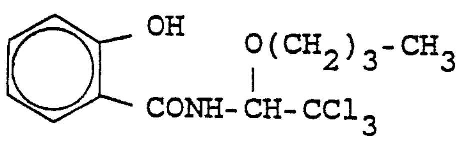

NK-483

Die Erfindung wird durch nachfolgende Beispiele erläutert:

A. Formulierungsbeispiele

Beispiel 1:

Ein Stäubemittel wird erhalten, indem man

10 Gewichtsteile Wirkstoff
und 90 Gewichtsteile Talkum

als Inertstoff mischt und in einer Schlagmühle zerkleinert.

Beispiel 2:

Ein in Wasser leicht dispergierbares, benetzbares Pulver wird erhalten, indem man

25 Gewichtsteile Wirkstoff
64 Gewichtsteile kaolinhaltigen Quarz als Inertstoff
10 Gewichtsteile ligninsulfonsaures Kalium
und 1 Gewichtsteil oleoylmethyltaurinsaures Natrium als Netz- und Dispergiermittel

mischt und in einer Stiftmühle mahlt.

Beispiel 3:

Ein in Wasser leicht dispergierbares Dispersionskonzentrat wird erhalten, indem man

20 Gewichtsteile Wirkstoff mit
6 Gewichtsteilen Alkylphenolpolyglykoläther (®Triton X 207)
3 Gewichtsteilen Isotridecanolpolyglykoläther (8 AeO)
und 71 Gewichtsteilen paraffinischem Mineralöl (Siedebereich z.B. ca. 255 bis über 377°C)

mischt und in einer Reibkugelmühle auf eine Feinheit von unter 5 Mikron vermahlt.

Beispiel 4:

Ein emulgierbares Konzentrat wird erhalten aus

15 Gewichtsteilen Wirkstoff
75 Gewichtsteilen Cyclohexanon als Lösungsmittel
und 10 Gewichtsteilen oxäthyliertes Nonylphenol (10 AeO) als Emulgator.

B. Chemische Beispiele

Beispiel 1:

2-(2-Chlorbenzyloxy-methyl)-1-(1-imidazolyl-carbamoyl)-piperidin

Zu 8,00 g (0,033 Mol) 2-(2-Chlorbenzyloxy-methyl)-piperidin gelöst in 50 ml Toluol gab man 5,50 g (0,034 Mol) und erwärmte 4 h auf 50°C. Nach dem Abkühlen wurde mit 2 x 30 ml Wasser gewaschen und das Solvens im Vakuum abdestilliert. Man erhielt 9,20 g (84 %) der Titelverbindung als leicht gelblich gefärbte Kristalle vom Smp. 91-2°C.

In analoger Weise lassen sich die Verbindungen der Formel I aus folgender Tabelle herstellen, wobei Y jeweils Wasserstoff ist.

| Beispiel Nr. | n | $(R)_m$ | Smp. (°C) |
|---|---|---|---|
| 2 | 5 | $2-CH_2OCH_2CH=CH-C_6H_5$ | Öl |
| 3 | 5 | $2-CH_2OCH_2-C(CH_3)=CHCH_2CH_2CH=C(CH_3)_2$ | Öl |
| 4 | 5 | $2-CH_2OCH_2C≡CH_2$ | |
| 5 | 5 | $2-CH_2O-cyclo-C_6H_{11}$ | |
| 6 | 5 | $3-CH_2O-C_6H_4-4-CF_3$ | |
| 7 | 5 | $3-O-C_6H_4-4-CF_3$ | Öl |
| 8 | 5 | $3-O-C_6H_3-2-Cl, 4-CF_3$ | |
| 9 | 5 | $4-O-C_6H_4-4-CF_3$ | 103-4 |
| 10 | 5 | $3-CH_2O-C_6H_4-4-CF_3$ | Öl |
| 11 | 5 | $3-CH_2O-CH_2C_6H_2-2,4,6-Cl_3$ | Öl |
| 12 | 5 | $3-CH_2O-C_6H_4-2-Cl,4-CF_3$ | Öl |
| 13 | 5 | $3-OC_6H_4-3-CF_3$ | |
| 14 | 5 | $3-OC_6H_2-2,4,6-Cl_3$ | |
| 15 | 5 | $3-OC_6H_3-2-Cl,4-CF_3$ | |
| 16 | 5 | $3-OCH_2-C_6H_2-2,4,6-Cl_3$ | Öl |
| 17 | 5 | $2-CH_2OC_6H_4-4-CF_3$ | Öl |
| 18 | 5 | $2-CH_2OC_6H_3-2-Cl,4-CF_3$ | Öl |
| 19 | 5 | $2-CH_2OC_6H_2-2,6-Cl_2-4-CF_3$ | Öl |
| 20 | 5 | $2-CH_2OC_6H_2-2,4,6-Cl_3$ | |
| 21 | 5 | $2-CH_2OC_6H_3-2,4-Cl_2$ | |
| 22 | 5 | $2-CH_2OC_6H_4-4-Cl$ | |
| 23 | 5 | $2-CH_2OC_6H_4-4-F$ | |
| 24 | 5 | $2-CH_2OC_6H_3-2,4-F_2$ | |
| 25 | 5 | $2-CH_2SC_6H_4-4-Cl$ | Öl |
| 26 | 5 | $2-CH_2O-C_6H_4-2-CF_3$ | Öl |
| 27 | 5 | $2-CH_2O-C_6H_3-3-Cl,4-CF_3$ | Öl |
| 28 | 5 | $2-CH_2OCH_2C_6H_2-2,4,6-Cl_3$ | Öl |
| 29 | 5 | $2-CH_2OCH(C_6H_5)_2$ | Öl |
| 30 | 5 | $2-CH_2CH_2O-C_6H_4-4-CF_3$ | Öl |
| 31 | 5 | $2-CH_2CH_2O-C_6H_4-2-CF_3$ | Öl |
| 32 | 5 | $2-CH_2CH_2O-C_6H_3-3-Cl,4-CF_3$ | Öl |
| 33 | 5 | $2-CH_2OC_6H_3-2-CN,3-F$ | Öl |

| Beispiel Nr. | n | $(R)_m$ | Smp. (°C) |
|---|---|---|---|
| 34 | 5 | $2\text{-}CH_2OCH_2C_6H_4\text{-}4Cl$ | Öl |
| 35 | 5 | $2\text{-}CH_2OCH_2C_6H_4\text{-}4\text{-}CF_3$ | Öl |
| 36 | 5 | $2\text{-}CH_2OCH[C_6H_4\text{-}4F][C_6H_3\text{-}2,4\text{-}Cl_2]$ | Öl |
| 37 | 5 | $2\text{-}CH(CH_3)\text{-}OC_6H_4\text{-}4\text{-}CF_3$ | Öl |
| 38 | 5 | $2\text{-}CH_2CH(CH_3)\text{-}O\text{-}C_6H_4\text{-}4\text{-}CF_3$ | Öl |
| 39 | 4 | $2\text{-}CH_2O\text{-}C_6H_3\text{-}2,6\text{-}Cl_2$ | Öl |
| 40 | 4 | $2\text{-}CH_2OCH_2\text{-}C_6H_2\text{-}2,4,6\text{-}Cl_3$ | Öl |
| 41 | 4 | $2\text{-}CH_2O\text{-}C_6H_3\text{-}2,6\text{-}Cl_2,4\text{-}CF_3$ | Öl |
| 42 | 4 | $2\text{-}CH_2O\text{-}C_6H_4\text{-}4\text{-}CF_3$ | Öl |
| 43 | 4 | $2\text{-}CH_2O\text{-}C_6H_4\text{-}2\text{-}CF_3$ | |
| 44 | 4 | $2\text{-}CH_2O\text{-}C_6H_4\text{-}2\text{-}Cl,4\text{-}CF_3$ | Öl |
| 45 | 5 | $2,3\text{-bis-}(CH_2O\text{-}C_6H_4\text{-}4CF_3)$ | |
| 46 | 5 | $2,6\text{-bis-}(CH_2O\text{-}C_6H_4\text{-}4\text{-}CF_3)$ | |
| 47 | 5 | $2\text{-}CH_2O\text{-}$(2-pyridyl-5-$CF_3$) | Öl |
| 48 | 5 | $2\text{-}CH_2O\text{-}$(2-pyridyl-3-Cl,5-$CF_3$) | Öl |
| 49 | 5 | $2\text{-}CH_2O\text{-}$(2-pyridyl-3-$CF_3$,5-Cl) | |
| 50 | 5 | $2\text{-}CH_2O\text{-}$(2-pyridyl) | 83-5 |
| 51 | 5 | $2\text{-}CH_2O\text{-}$(2-pyridyl-5-Br) | 97-101 |
| 52 | 5 | $2\text{-}CH_2O\text{-}$(2-pyridyl-6-Cl) | 67-75 |
| 53 | 5 | $2\text{-}CH_2CH_2O\text{-}$(2-pyridyl) | Öl |
| 54 | 5 | $2\text{-}CH_2CH_2O\text{-}$(2-pyridyl-5-Br) | Öl |
| 55 | 5 | $2\text{-}CH_2CH_2O\text{-}$(2-pyridyl-5-Cl) | 70-4 |
| 56 | 5 | $2\text{-}CH_2CH_2O\text{-}$(2-pyridyl-5,6-$(CH_3)_2$) | 64-6 |
| 57 | 5 | $2\text{-}CH_2CH_2O\text{-}$(2-pyridyl-6-Cl) | Öl |
| 58 | 5 | 4-O-(2-pyridyl-6-Cl) | 149-52 |
| 59 | 5 | 4-O-(2-pyridyl) | 80-2 |
| 60 | 6 | $2\text{-}CH_2O\text{-}C_6H_4\text{-}4\text{-}CF_3$ | |
| 61 | 5 | $2\text{-}CH_2O\text{-}$(6-Cl-benzoxazol-2-yl) | |
| 62 | 5 | $2\text{-}CH_2O\text{-}$(6-Cl-benzthiazol-2-yl) | |

| Beispiel Nr. | n | $(R)_m$ | Smp. (°C) |
|---|---|---|---|
| 63 | 5 | 2-$CH_2O$- 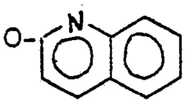 | |
| 64 | 5 | 2-$CH_2O$- 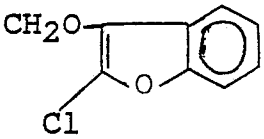 | |
| 65 | 3 | 2-$CH_2OC_6H_4$-4-$CF_3$ | |
| 66 | 6 | 2-$CH_2OC_6H_4$-4-$CF_3$ | |
| 67 | 5 | 2-$CH_2OCH_2$-$C_6H_3$-2-F,4-Cl | Öl |
| 68 | 5 | 2-$CH_2OCH_2$-$OC_6H_4$-2-Cl | Öl |
| 69 | 5 | 2-$CH_2OCH_2$-$C_6H_4$-4-F | Öl |
| 70 | 5 | 2-$CH_2CH(CH_3)O$-$C_6H_3$-3-Cl,4-$CF_3$ | Öl |
| 71 | 5 | 3-$OC_6H_3$-3-Cl,4-$CF_3$ | Öl |
| 72 | 5 | 2-$CH_2OCH_2$-$C_6H_3$-3,4-$Cl_2$ | 82-4 |
| 73 | 5 | 2-$CH_2OCH_2$-$C_6H_3$-2,4-$Cl_2$ | 110-2 |
| 74 | 4 | 2-$CH_2O$-$C_6H_3$-2-F,4-$CF_3$ | Öl |
| 75 | 5 | 2-$CH_2O$-$C_6H_3$-2-F,4-$CF_3$ | Öl |
| 76 | 5 | 2-$CH_2OCH_2$- 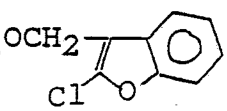 | Öl |
| 77 | 5 | 2-$CH_2OCH_2CH=CHCH_3$ | Öl |
| 78 | 5 | 2-$CH_2OCH_2CH=CCl$-$C_6H_3$-2,4-$Cl_2$ | Öl |
| 79 | 5 | 2-$CH_2OCH_2CH=CCl$-$C_6H_4$-4-Cl | Öl |
| 80 | 5 | 2-$CH_2OCH_2CH=CCl$-$C_6H_5$ | Öl |
| 81 | 5 | 2-$CH_2OCH(CH_3)$-$C_6H_5$ | Öl |
| 82 | 5 | 2-$CH_2OCH_2O=CCl$-$C_6H_4$-4-$CH_3$ 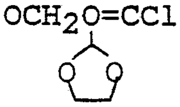 | Öl |
| 83 | 5 | 2-$CH_2$-$OCH_2C_6H_3$-3,5-$Cl_2$ | Öl |
| 84 | 5 | 2-$CH_2OCH_2$-1-naphthyl | Öl |

| Beispiel Nr. | n | $(R)_m$ | Smp. (°C) |
|---|---|---|---|
| 85 | 5 | $2\text{-}CH_2OCH_2C(CH_3)=CH_2$ | Öl |
| 86 | 5 | $2\text{-}CH_2OC_4H_9(n)$ | Öl |
| 87 | 5 | $2\text{-}CH_2OCOCH=CH\text{-}C_6H_5$ | Öl |
| 88 | 5 | $2\text{-}CH_2CH_2OC_6H_3\text{-}2Cl\text{-}4\text{-}CF_3$ | Öl |
| 89 | 5 | $2\text{-}CH_2CH_2OC_6H_3\text{-}2F\text{-}4\text{-}CF_3$ | Öl |
| 90 | 5 | $2\text{-}CH_2CH_2OCH_2CH=CH\text{-}C_6H_5$ | Öl |
| 91 | 5 | $2\text{-}CH_2CH_2O$(-2-pyridyl-3-Cl-5-$CF_3$) | Öl |
| 92 | 5 | $2\text{-}CH_2CH(CH_3)OCH_2\text{-}C_6H_4\text{-}4\text{-}F$ | Öl |
| 93 | 5 | $2\text{-}CH_2CH(CH_3)OCH_2C_6H_2\text{-}2,4,6\text{-}Cl_3$ | Öl |
| 94 | 5 | $2\text{-}CH_2CH_2OCH_2C_6H_4\text{-}4\text{-}Cl$ | Öl |
| 95 | 5 | $2\text{-}CH_2CH_2OCH_2C_6H_3\text{-}2\text{-}F,4\text{-}Cl$ | Öl |
| 96 | 5 | $2\text{-}CH_2CH_2OCOC_6H_5$ | Öl |
| 97 | 5 | $2\text{-}CH_2OC_6H_3\text{-}2\text{-}CF_3,4\text{-}Cl$ | Öl |
| 98 | 5 | $2\text{-}CH_2CH_2OC_6H_3\text{-}2\text{-}CF_3,4\text{-}Cl$ | Öl |
| 99 | 5 | $2\text{-}CH_2CH_2OC_6H_2,2\text{-}Cl,4\text{-}CF_3,6\text{-}F$ | Öl |
| 100 | 5 | $2\text{-}CH_2CH_2OC_6H_2\text{-}2,6\text{-}Cl_2,4\text{-}CF_3$ | Öl |
| 101 | 5 | $2\text{-}CH_2CH_2OCH_2OC_6H_3\text{-}2,4\text{-}Cl_2$ | Öl |
| 102 | 5 | $2\text{-}CH_2CH_2OCH_3$-1-naphthyl | Öl |
| 103 | 5 | $2\text{-}CH_2CH(CH_3)O\text{-}C_6H_4\text{-}2\text{-}Cl,4\text{-}CF_3$ | |
| 104 | 5 | $2\text{-}CH_2CH_2OCOCH=CH\text{-}C_6H_5$ | |
| 105 | 5 | $2\text{-}CH_2CH_2O$-(2-chinolyl) | |
| 106 | 5 | $2\text{-}CH_2CH_2O$-(2-chinoxalinyl-6-Cl) | |

C. Beispiele der biologischen Wirkung

Beispiel 1

Filterpapierstreifen (10 mm breit, 90 mm lang) werden mit den formulierten Wirkstoffen der Formel I mit einer Konzentration von 500 ppm Wirksubstanz gleichmäßig benetzt (ca. 200 µl/Streifen) und auf ein je nach Pilzart unterschiedliches Agar-Medium aufgelegt. Dem Agar werden zuvor in noch flüssigem Zustand je Petrischale 0,5 ml Suspensionskultur des Testorganismus (ca. $10^5$ - $10^6$ Konidien/ml) zugegeben und die so behandelten Agarplatten anschließend bei 25°C bebrütet. Nach 3 - 4-tägiger Inokulation mißt man die Inhibitionszonen aus und bewertet die Wirkung der Prüfsubstanzen anhand der ausgebildeten Hemmzonen.

Tabelle 1 Botrytis cinerea:
BCM- und Iprodion-sensibler (s) und -resistenter (r) Stamm

Verbindungen Hemmzonen in mm bei 500 ppm Wirkstoff gemäß

| Beispiel | s | r |
|---|---|---|
| 17 | 30 | 22 |
| 27 | 30 | 26 |
| Kontrolle | 0 | 0 |

Beispiel 2

Ackerbohnen werden bei ca. 26 - 28°C und 60 % relativer Luftfeuchte angezogen. 14 Tage nach der Aussaat (Wuchshöhe 13 - 16 cm) sind die Pflanzen für die Versuche geeignet.

Nach Vorbereitung der Pflanzen für den Versuch erfolgt die Applikation der Versuchspräparate in einer Konzentration von 500 ppm mit einem Glaszerstäuber bei 0,3 - 0,5 bar Überdruck auf die Blätter der Ackerbohnen. Die behandelten Pflanzen werden zum Trocknen abgestellt und ca. 3 h später inokuliert.

Mit den frischen Konidien werden Sporensuspensionen mit 4 x $10^5$ Sporen pro 1 ml hergestellt. Anschließend werden mit Hilfe eines fein sprühenden Glaszerstäubers die Sporensuspensionen gleichmäßig auf die Vicia faba Pflanzen appliziert und die Pflanzen in eine Klimakammer bei 20 - 22°C und ca. 99 % relativer Luftfeuchte aufgestellt. Die Infektion der Pflanzen äußert sich in der Bildung schwarzer Flecken auf Blättern und Stengeln, die bei starkem Befall die Pflanzen zusammenbrechen läßt.
Die Auswertung der Versuche erfolgt 3 bzw. 6 Tage nach Inokulation.
Der Wirkungsgrad der Wirkstoffe wird ausgedrückt in % im Vergleich zur unbehandelten, infizierten Kontrolle.

Tabelle II

| Verbindungen gemäß Beispiel | Wirkungsgrad in % bei 500 ppm Wirkstoff BCM- und Iprodion-sensibler (s) und resistenter (r) Stamm | |
|---|---|---|
| | s | r |
| 17 | 100 | 100 |
| 27 | 100 | 100 |
| Kontrolle | 0 | 0 |

**Beispiel 3**

Weinsämlinge der Sorten "Riesling/Ehrenfelder" wurden ca. 6 Wochen nach der Aussat mit wäßrigen Suspensionen der beanspruchten Verbindungen tropfnaß behandelt. Die Anwendungskonzentrationen betrugen 500 und 250 mg Wirkstoff pro Liter Spritzbrühe.

Nach dem Antrocknen des Spritzbelages wurden die Pflanzen mit einer Zoosporangiensuspension von Plasmopara viticola inokuliert und tropfnaß in eine Klimakammer bei einer Temperatur von ca. 23°C und einer Luftfeuchtigkeit von ca. 80 - 90 % gebracht.

Nach einer Inkubationszeit von 7 Tagen wurden die Pflanzen über Nacht in die Klimakammer gestellt und die Krankheit zum Ausbruch gebracht. Anschließend erfolgte die Befallauswertung. Der Befallsgrad wurde in % befallener Blattfläche im Vergleich zu den unbehandelten, infizierten Kontrollpflanzen ausgedrückt und ist in Tabelle III wiedergegeben.

**Tabelle III**

| | mit Plasmopara viticola befallene Blattfläche in % bei mg Wirkstoff/Liter Spritzbrühe | |
|---|---|---|
| Bsp. Nr. | 500 | 250 |
| 18 | 0 | 0 |
| unbehandelte infizierte Pflanzen | 100 | |

**Beispiel 4**

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 1 Tag nach Inokulation werden die Pflanzen mit den in Tabelle IV aufgeführten Verbindungen in den Wirkstoffkonzentrationen gleichmäßig benetzt. Nach einer Inkubationszeit von 10 Tagen werden die Pflanzen auf den Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle IV zusammengefaßt.

**Tabelle IV**

| | mit Weizenmehltau befallene Blattfläche in % bei mg Wirkstoff/Liter Spritzbrühe | | | | |
|---|---|---|---|---|---|
| Bsp. Nr. | 500 | 250 | 125 | 60 | 30 |
| 53 | 0 | 0 | 0 | 0 | 0 |
| 54 | 0 | 0 | 0 | 0 | 0 |
| 55 | 0 | 0 | 0 | 0 | 0 |
| 52 | 0 | 0 | 0 | 0 | 0 |
| 17 | 0 | 0 | 0 | 0 | 0 |
| 27 | 0 | 0 | 0 | 0 | 0 |
| 88 | 0 | 0 | 0 | 0 | 0 |
| 30 | 0 | 0 | 0 | 0 | 0 |
| 35 | 0 | 0 | 0 | 0 | 0 |
| 7 | 0 | 0 | 0 | 0 | 0 |
| 89 | 0 | 0 | 0 | 0 | 0 |
| 91 | 0 | 0 | 0 | 0 | 0-3 |
| 94 | 0 | 0 | 0 | 0 | 0 |
| 95 | 0 | 0 | 0 | 0 | 0-3 |
| unbehandelte, infizierte Pflanzen | | 100 | | | |

**Beispiel 5**

Gerstenpflanzen (Igri) werden im 3-Blattstadium mit Konidien des Gerstenmehltaus (Erysiphe graminis hordei) stark inokuliert und in einem Gewächshaus bei 20°C und einer relativen Luftfeuchte von 40 - 60 % aufgestellt. 1 Tag nach Inokulation werden die Pflanzen mit den in Tabelle V aufgeführten Verbindungen in den angegebenen Wirkstoffkonzentrationen gleichmäßig benetzt. Nach einer Inkubationszeit von 10 Tagen werden die Pflanzen auf Befall mit Gerstenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle V zusammengefaßt.

**Tabelle V**

| | mit Gerstenmehltau befallene Blattfläche in % bei mg Wirkstoff/Liter Spritzbrühe | | |
|---|---|---|---|
| Bsp. Nr. | 500 | 250 | 125 |
| 51 | 0 | 0 | 0-3 |
| 54 | 0 | 0 | 0 |
| 55 | 0 | 0 | 0 |
| 52 | 0 | 0 | 0-3 |
| 30 | 0 | 0 | 0-3 |
| 94 | 0 | 0-3 | 0-3 |
| 95 | 0 | 0 | 0-3 |
| unbehandelte, infizierte Pflanzen | | | 100 |

**Beispiel 6**

Gurkenpflanzen (Sorte Delikateß) werden im 2-Blattstadium mit einer Konidiensuspension von Gurkenmehltau (Erysiphe cichoracearum) stark inokuliert. Nach einer Abtrocknungszeit der Sporensuspension von 30 Minuten werden die Pflanzen in einem Gewächshaus bei 22°C und 90 % relativer Luftfeuchte aufgestellt.

3 Tage nach Infektion werden die Pflanzen mit den in Tabelle VI genannten Verbindungen und Wirkstoffkonzentrationen tropfnaß gleichmäßig benetzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis ist in Tabelle VI zusammengefaßt.

**Tabelle VI**

| | mit Gurkenmehltau befallene Blattfläche in % bei mg Wirkstoff/Liter Spritzbrühe | | | | |
|---|---|---|---|---|---|
| Bsp. Nr. | 500 | 250 | 125 | 60 | 30 |
| 26 | 0 | 0 | 0 | 0 | 0-3 |
| 88 | 0 | 0 | 0 | 0 | 0 |
| 35 | 0 | 0 | 0 | 0 | 0 |
| 31 | 0 | 0 | 0 | 0 | 0-3 |
| 77 | 0 | 0 | 0 | 0 | 0-3 |
| 89 | 0 | 0 | 0 | 0 | 0 |
| 91 | 0 | 0 | 0 | 0 | 0 |
| unbehandelte, infizierte Pflanzen | | 100 | | | |

Beispiel 7

Ca. 6 Wochen alte Apfelsämlinge ("Malus commuis") wurden mit den in Tabelle VII angegebenen Wirkstoffkonzentrationen gleichmäßig benetzt. Nach dem Antrocknen der Spritzbrühe wurden die Pflanzen mit einer Sporensuspension von Venturia inaequalis gleichmäßig tropfnaß benetzt und für 48 Stunden in eine dunkel gehaltene Klimakammer bei einer Temperatur von 20°C und 100 % rel. Luftfeuchte gestellt.

Anschließend wurden die Pflanzen in ein Gewächshaus mit einer Temperatur von 15 - 17°C und ca. 100 % rel. Luftfeuchte gebracht. Ca. 2 Wochen nach der Inokulation erfolgte die Befallauswertung. Der Befallsgrad der Pflanzen mit Apfelschorf wurde in % befallener Blattfläche, bezogen auf unbehandelte, infizierte Pflanzen ausgedrückt und ist in Tabelle VII wiedergegeben.

**Tabelle VII**

| | % Schorfbefall bei mg Wirkstoff/Liter Spritzbrühe | | | |
|---|---|---|---|---|
| Bsp. Nr. | 500 | 250 | 125 | 60 |
| 54 | 0 | 0 | 0 | 0 |
| 30 | 0 | 0 | 0 | 0 |
| 35 | 0 | 0 | 0 | 0 |
| 2 | 0 | 0 | 0 | 0 |
| 38 | 0 | 0 | 0 | 0 |
| 7 | 0 | 0 | 0 | 0 |
| 32 | 0 | 0 | 0 | 0 |
| 90 | 0 | 0 | 0 | 0-3 |
| unbehandelte, infizierte Pflanzen | 100 | | | |

**Beispiel 8**

Weizenpflanzen ("Jubilar") werden im 2-Blattstadium mit den in Tabelle VIII angegebenen Wirkstoffkonzentrationen gleichmäßig benetzt. Nach dem Antrocknen der Spritzbrühe wurden die Pflanzen mit einer Sporensuspension von Leptosphaeria nodorum gleichmäßig tropfnaß benetzt und für 48 Stunden in eine dunkel gehaltene Klimakammer bei einer Temperatur von 25°C und 100 % rel. Luftfeuchte gestellt.

Anschließend wurden die Pflanzen in ein Gewächshaus mit einer Temperatur von 22 - 25°C und ca. 90 % rel. Luftfeuchte gebracht. Ca. 1 Woche nach der Inokulation erfolgte die Befallsauswertung. Der Befallsgrad wird ausgedrückt in % befallener Blattfläche, bezogen auf unbehandelte, infizierte Kontrollpflanzen (= 100 % Befall): Das Ergebnis ist in Tabelle VIII zusammengefaßt.

**Tabelle VIII**

| | mit Leptosphaeria nodorum befallene Blattfläche in % bei mg Wirkstoff/Liter Spritzbrühe | | |
|---|---|---|---|
| Bsp. Nr. | 500 | 250 | 125 |
| 17 | 0 | 0 | 0 |
| 27 | 0 | 0 | 0 |
| 88 | 0 | 0 | 0 |
| 30 | 0 | 0 | 0 |
| 35 | 0 | 0 | 0 |
| 94 | 0 | 0 | 0 |
| 95 | 0 | 0 | 0 |
| unbehandelte, infizierte Pflanzen | | 100 | |

## Beispiel 9

Ackerbohnen (Sorten "Herz Freya" oder "Frank's Ackerperle") werden bei ca. 20°C und 60 % rel. Luftfeuchte angezogen. 14 Tage nach Aussaat (Wuchshöhe 13 - 16 cm) sind die Pflanzen für die Versuche geeignet.

Nach Vorbereitung der Pflanzen für den Versuch erfolgt die Applikation der Versuchspräparate mit den in Tabelle IX angegebene Konzentrationen. Die behandelten Pflanzen werden zum Trocknen abgestellt und ca. 3 h später inokuliert.

Mit den frischen Konidien werden Sporensuspensionen mit 1,5 x $10^6$ Sporen pro 1 ml hergestellt. Anschließend wird mit Hilfe eines fein sprühenden Glaszerstäubers die Sporensuspension gleichmäßig auf die Vicia faba-Pflanzen appliziert und die Pflanzen in eine Klimakammer bei 20 - 22°C und ca. 90 % rel. Luftfeuchte aufgestellt. Die Infektion der Pflanzen äußert sich in der Bildung schwarzer Flecken auf den Blättern und Stengeln, die bei starkem Befall die Pflanzen zusammenbrechen läßt. Die Auswertung der Versuche erfolgt ca. 1 Woche nach Inokulation.

Der Wirkungsgrad der Prüfsubstanzen wird prozentual zur unbehandelten, infizierten Kontrolle ausgedrückt.

**Tabelle IX**

| Bsp. Nr. | Wirkungsgrad in % bei mg Wirkstoff/Liter Spritzbrühe BCM- und Iprodion-sensibler (s) und resistenten(r) Stamm | | | | | |
|---|---|---|---|---|---|---|
| | (s) | | | (r) | | |
| | 500 | 250 | 125 | 500 | 250 | 125 |
| 17 | 97 | 95 | 85 | 95 | 90 | 85 |
| 27 | 90 | 90 | 90 | 95 | 95 | 90 |

**Tabelle IX (Fortsetzung)**

| Bsp. Nr. | Wirkungsgrad in % bei mg Wirkstoff/Liter Spritzbrühe BCM- und Iprodion-sensibler (s) und resistenten(r) Stamm | | | | | |
|---|---|---|---|---|---|---|
| | (s) | | | (r) | | |
| | 500 | 250 | 125 | 500 | 250 | 125 |
| 30 | 85 | 85 | 85 | 85 | 85 | 85 |
| 90 | 90 | 85 | 85 | 90 | 85 | 85 |
| unbehandelte, infizierte Pflanzen | 0 | 0 | 0 | 0 | 0 | 0 |

## Patentansprüche

1. Verbindungen der Formel (I)

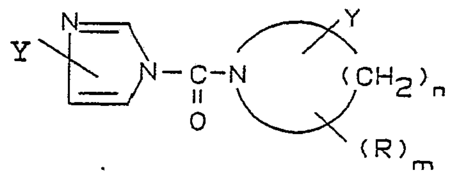

(I)

worin bedeuten:

n = 2, 3, 4, 5 oder 6,

m = 1 oder 2,

R = $-OR^1$, $-\underset{R^2}{CH}O-R^1$, $-\underset{R^2}{CH}-SR^1$, $-CH_2\underset{R^2}{CH}OR^1$ oder $-CH_2CH_2CH_2OR^1$,

$R^1$= $(C_1-C_{10})$-Alkyl, $(C_3-C_{10})$-Alkenyl, die beide durch 1 bis 6 Fluor-, Chlor- oder Bromatome, Hydroxy, $C_1-C_4$-Alkoxy, Phenyl oder Phenoxy, die beide unsubstituiert oder durch Halogen, $(C_1-C_4)$Alkyl, $(C_1-C_4)$Alkoxy oder $CF_3$ substituiert sind, substituiert sein können, $C_3$-Alkinyl, $(C_3-C_6)$-Cycloalkyl, $(C_5-C_6)$-Cycloalkenyl, Phenyl, Benzyl, Benzhydryl, Trityl, Biphenyl, Phenoxyphenyl, Phenylthiophenyl, Naphthyl, 1,2,3,4-Tetrahydronaphthyl, Indanyl, Fluorenyl, Pyridyl, Benzthiazolyl, Benzoxazolyl, Chinolinyl, Thiochromanyl, Benzofuranylmethyl, Chinolinylmethyl, Phenacyl, wobei die genannten Ringsysteme unsubstituiert oder mit 1 - 5 Substituenten aus der Gruppe Halogen, $(C_1-C_8)$-Alkyl, Halo$(C_1-C_8)$alkyl, $(C_3-C_8)$-Cycloalkyl, $(C_1-C_8)$-Alkoxy, Halo$(C_1-C_8)$alkoxy, $(C_3-C_8)$-Cycloalkyl-$(C_1-C_4)$alkyl, $(C_1-C_8)$Alkylthio, Halo$(C_1-C_4)$alkylthio, $(C_1-C_8)$-Alkylsulfinyl-, $(C_1-C_8)$Alkylsulfonyl, $NO_2$, -CN, -CHO oder $(C_1-C_4)$Alkylcarbonyl substituiert sind; $(C_4-C_{10})$Alkadienyl,

$R^2$= H oder $(C_1-C_4)$Alkyl und

Y = unabhängig voneinander H, $(C_1-C_4)$Alkyl, Phenyl oder Halogenphenyl sowie deren Metallsalzkomplexe.

2. Verbindungen der Formel I von Anspruch 1, worin Y = H, n = 4 oder 5, m = 1, $R=OR^1$, $CH_2OR^1$ oder $CH_2CH_2OR^1$ und $R^1$ = Phenyl, Benzyl, Benzhydryl, Biphenyl, Pyridyl oder Chinolyl bedeutet, wobei die genannten Ringsysteme unsubstituiert oder mit 1 - 3 Substituenten aus der Gruppe F, Cl, Br, $(C_1-C_3)$-Alkyl, Halo$(C_1-C_3)$alkyl, $(C_1-C_3)$-Alkoxy, Halo$(C_1-C_3)$alkoxy, $(C_1-C_3)$-Alkylthio, $CF_3S$, $CF_3CH_2S$, $NO_2$, CN, CHO oder $-COCH_3$ substituiert sind, sowie deren Metallsalzkomplexe.

3. Verfahren zur Herstellung einer Verbindung I nach Ansprüchen 1 oder 2, dadurch gekennzeichnet, daß man

a) eine Verbindung der Formel (II)

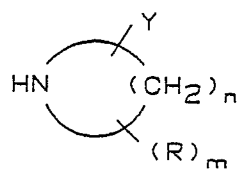

(II)

mit einem N,N'- Carbonyldiimidazol der Formel (III)

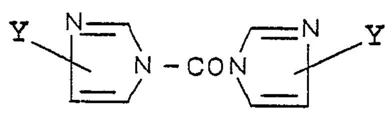

(III)

oder

b) eine Verbindung der Formel (IV)

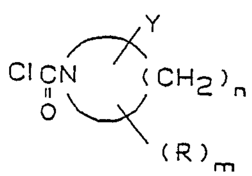

(IV)

mit einer Imidazolverbindung der Formel (V)

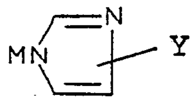 (V)

worin M = ein Alkalimetall bedeutet, umsetzt und die erhaltene Verbindung gegebenenfalls in einen Metallkomplex überführt werden.

4. Fungizide Mittel, dadurch gekennzeichnet, daß sie eine Verbindung der Formel I von Ansprüchen 1 oder 2 enthalten.

5. Verwendung von Verbindung der Formel I zur Bekämpfung von Schadpilzen.

6. Verfahren zur Bekämpfung von Schadpilzen, dadurch gekennzeichnet, daß man auf diese, die zu schützenden Pflanzen, Anbauflächen oder Substrate eine wirksame Menge einer Verbindung der Formel I von Ansprüchen 1 oder 2 appliziert.

0 270 074

Patentansprüche Spanien

1. Verfahren zur Herstellung von Verbindungen der Formel (I)

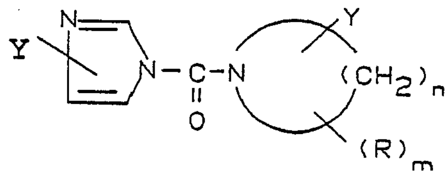

(I),

worin bedeuten:

n = 2, 3, 4, 5 oder 6,

m = 1 oder 2,

R = $-OR^1$, $-\underset{R^2}{CH}O-R^1$, $-\underset{R^2}{CH}-SR^1$, $-CH_2\underset{R^2}{C}HOR^1$ oder $-CH_2CH_2CH_2OR^1$,

$R^1$= $(C_1-C_{10})$-Alkyl, $(C_3-C_{10})$-Alkenyl, die beide durch 1 bis 6 Fluor-, Chlor- oder Bromatome, Hydroxy, $C_1-C_4$-Alkoxy, Phenyl oder Phenoxy, die beide unsubstituiert oder durch Halogen, $(C_1-C_4)$Alkyl, $(C_1-C_4)$Alkoxy oder $CF_3$ substituiert sind, substituiert sein können, $C_3$-Alkinyl, $(C_3-C_6)$-Cycloalkyl, $(C_5-C_6)$-Cycloalkenyl, Phenyl, Benzyl, Benzhydryl, Trityl, Biphenyl, Phenoxyphenyl, Phenylthiophenyl, Naphthyl, 1,2,3,4-Tetrahydronaphthyl, Indanyl, Fluorenyl, Pyridyl, Benzthiazolyl, Benzoxazolyl, Chinolinyl, Thiochromanyl, Benzofuranylmethyl, Chinolinylmethyl, Phenacyl, wobei die genannten Ringsysteme unsubstituiert oder mit 1 - 5 Substituenten aus der Gruppe Halogen, $(C_1-C_8)$-Alkyl, Halo$(C_1-C_8)$alkyl, $(C_3-C_8)$-Cycloalkyl, $(C_1-C_8)$-Alkoxy, Halo$(C_1-C_8)$alkoxy, $(C_3-C_8)$-Cycloalkyl-$(C_1-C_4)$alkyl, $(C_1-C_8)$Alkylthio, Halo$(C_1-C_4)$alkylthio, $(C_1-C_8)$-Alkylsulfinyl-, $(C_1-C_8)$Alkylsulfonyl, $NO_2$, -CN, -CHO oder $(C_1-C_4)$Alkylcarbonyl substituiert sind; $(C_4-C_{10})$Alkadienyl,

$R^2$= H oder $(C_1-C_4)$Alkyl und

Y = unabhängig voneinander H, ($C_1$-$C_4$)Alkyl, Phenyl oder Halogenphenyl sowie deren Metallsalzkomplexe, dadurch gekennzeichnet, daß man

a) eine Verbindung der Formel (II)

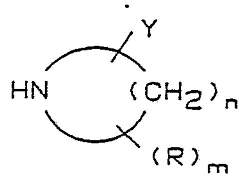

(II)

mit einer Verbindung der Formel (III)

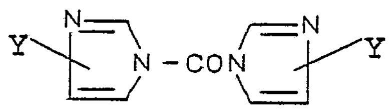

(III)

oder

b) eine Verbindung der Formel (IV)

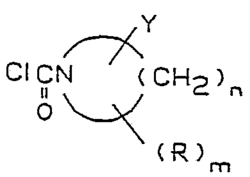

(IV)

mit einer Imidazolverbindung der Formel (V)

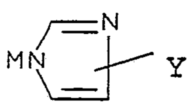

(V)

worin M = ein Alkalimetall bedeutet, umsetzt und die erhaltene Verbindung gegebenenfalls in einen Metallkomplex überführt werden.

2. Verfahren gemäß Anspruch 1, dadurch gekennzeichnet, daß in Formel I Y = H, n = 4 oder 5, m = 1, R $OR^1$, $CH_2OR^1$ oder $CH_2CH_2OR^1$ und $R^1$ = Phenyl, Benzyl, Benzhydryl, Biphenyl, Pyridyl oder Chinolyl bedeutet, wobei die genannten Ringsysteme unsubstituiert oder mit 1 - 3 Substituenten aus der Gruppe F, Cl, Br, $(C_1-C_3)$-Alkyl, Halo$(C_1-C_3)$alkyl, $(C_1-C_3)$-Alkoxy, Halo$(C_1-C_3)$alkoxy, $(C_1-C_3)$-Alkylthio, $CF_3S$, $CF_3CH_2S$, $NO_2$, CN, CHO oder $-COCH_3$ substituiert sind, sowie deren Metallsalzkomplexe.

3. Fungizide Mittel, dadurch gekennzeichnet, daß sie eine Verbindung der Formel I von Ansprüchen 1 oder 2 enthalten.

4. Verwendung von Verbindung der Formel I zur Bekämpfung von Schadpilzen.

5. Verfahren zur Bekämpfung von Schadpilzen, dadurch gekennzeichnet, daß man auf diese, die zu schützenden Pflanzen, Anbauflächen oder Substrate eine wirksame Menge einer Verbindung der Formel I von Ansprüchen 1 oder 2 appliziert.